# EUROPEAN PATENT SPECIFICATION

(11) **EP 4 125 733 B1**
(45) Date of publication and mention of the grant of the patent: **27.08.2025**
(21) Application number: 21722311.4
(22) Date of filing: 30.03.2021
(51) Int. Cl.: A61F 2/30, A61B 17/17

(54) **PROSTHETIC DEVICE FOR TEMPOROMANDIBULAR JOINT AND CORRESPONDING PROSTHETIC ASSEMBLY**
PROTHESE FÜR KIEFERGELENK UND ENTSPRECHENDE PROTHESE
DISPOSITIF PROTHÉTIQUE POUR ARTICULATION TEMPORO-MANDIBULAIRE ET ENSEMBLE PROTHÉTIQUE CORRESPONDANT

(30) Priority: 03.04.2020 IT 202000007201
(43) Date of publication of application: 08.02.2023
(73) Proprietor: Università Degli Studi di Udine, 33100 Udine (IT); Azienda Sanitaria Universitaria Friuli Centrale, 33100 Udine (IT)
(72) Inventor: ROBIONY, Massimo, 33100 UDINE (IT); SEMBRONIO, Salvatore, 33100 UDINE (IT)
(74) Representative: Petraz, Gilberto Luigi
(86) International application number: PCT/IT2021/050089
(87) International publication number: WO 2021/199093

(56) References cited:
- EP-A1- 0 628 293
- WO-A1-2017/195022
- GB-A- 2 480 682
- US-A1- 2015 182 340

## Description

### FIELD OF THE INVENTION

The present invention concerns a prosthetic device for temporomandibular joint and a corresponding prosthetic assembly comprising said prosthetic device and a guide device for the preparation of a suitable bone seating.

The prosthetic device as above is particularly suitable for applications in patients with non-extensive joint pathologies, for example in the case of osteoarthrosis/osteoarthritis, condylar resorption, benign neoplasms, or in the case where previous surgeries have failed.

This solution can also be adopted as a first surgery in a large number of patients, where the traditional path would involve other attempts at functional arthroplasty, normally destined to fail.

### BACKGROUND OF THE INVENTION

It is known that the reconstruction of the temporomandibular joint is a problem due to the complex role it plays inside the stomatognathic system.

It plays an essential role in chewing, in speech, in supporting respiratory exchanges and in swallowing, and is a secondary growth center for the jaw during pre-puberty. Furthermore, the temporomandibular joint is subjected to repeated loading/unloading cycles more than any other joint in the body.

Given the complexity of the anatomy and biomechanics of said joint, surgery to solve problems and related pathologies is complex and currently very invasive.

The temporomandibular joint articulates the mandibular bone with the temporal bone, in particular it connects the mandibular condyle with the glenoid fossa of the temporal bone.

Prosthetic devices for the temporomandibular joint therefore comprise two components: a condyle prosthesis and a glenoid fossa prosthesis respectively associated, during use, with the mandibular condyle and the glenoid fossa of the temporal bone. Both components are made according to the specific needs of the patient and his/her anatomical morphology.

Known prosthetic devices for the temporomandibular joint are disclosed for example in US 2015/182340 A1, disclosing a prosthetic device for temporomandibular joint according to the preamble of claim 1, and GB 2 480 682 A, disclosing a guide device for preparing an anchoring seating for a prosthetic device, according to the preamble of claim 9.

Before they are applied, it is necessary to perform osteotomies to prepare the condyle and the glenoid fossa to accommodate the respective prostheses. For this purpose, guide devices can be provided able to facilitate the operation of bone resection performed by the surgeon. Furthermore, it is necessary to use traditional surgical burrs that cause damage, even very extensive, to the affected bone tissues and those surrounding them.

The prosthetic devices currently used are very bulky and require a very large installation space between the mandibular branch and the base of the skull, which is necessary to be able to insert the fossa prosthesis and the condyle prosthesis. Because of this, often the osteotomy of the condyle, and if necessary also of the glenoid fossa, must necessarily be very large and invasive.

This limits the indications for the implantation of the joint prosthesis only to very severe cases.

The sizes of current prostheses also make the surgical procedure very invasive, which provides a pre-auricular incision, for the insertion of the glenoid fossa prosthesis, and a retro-submandibular incision, for the insertion of the branch/condyle prosthesis.

The invasiveness of current prostheses and the surgical procedure for their installation lead to long hospitalization times and possible complications for the patient.

There is therefore a need to perfect a prosthetic device for application to the temporomandibular joint and corresponding prosthetic assembly that can overcome at least one of the disadvantages of the state of the art.

In particular, one purpose of the present invention is to provide a prosthetic device for temporomandibular joint the implantation of which requires a limited osteotomy of the condyle and possibly of the glenoid fossa.

Another purpose of the present invention is to provide a prosthetic device for temporomandibular joint that allows to extend the surgical indications, and therefore applicability, to a greater number of patients compared with the use of traditional prostheses.

Another purpose of the present invention is to provide a prosthetic device for temporomandibular joint that consists of a limited number of components so as to simplify and speed up its installation.

Another purpose of the present invention is to provide a prosthetic assembly for temporomandibular joint that is advantageously customized/individualized and patient-specific, and that allows to simplify and at the same time make the corresponding surgical technique less invasive.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a prosthetic device for temporomandibular joint, which overcomes the limits of the state of the art and eliminates the defects present therein, comprises a first prosthetic component, able to be associated with a mandibular condyle of a patient, and a coordinated second prosthetic component, able to be associated with a respective glenoid fossa of the patient.

The first prosthetic component is configured to cooperate with the second prosthetic component to define the temporomandibular joint and comprises a convex portion, configured to cooperate with the second prosthetic component to define the temporomandibular joint.

According to one aspect of the present invention, the first prosthetic component comprises at least one anchoring element projecting from, and attached at least to, the concave portion as above, since the anchoring element is contained in the concave portion and has a main extension along a latero-medial axis. The anchoring element is configured to be inserted into a mating anchoring seating present, or provided, on the mandibular condyle as above. Furthermore, the coupling seating is at least partly open along the latero-medial axis.

In this way, the first prosthetic component can be easily inserted in a direction parallel to the latero-medial axis as above, allowing to limit the removal of bone material from the patient to a minimum, and at the same time allows to greatly simplify the surgical practice. In addition, the minimal invasiveness of the surgical approach and of the resection of the condyle allows to extend the surgical indications and therefore a considerably greater applicability compared with the use of traditional prostheses.

In some embodiments, the first prosthetic component comprises a concave portion, defining a coupling seating having a shape mating with the shape of the mandibular condyle. The concave portion is therefore configured to cooperate with the second prosthetic component to define the temporomandibular joint. The convex portion as above is opposite the concave portion.

In accordance with some embodiments, a guide device is provided to prepare an anchoring seating for the prosthetic device as above, in particular to position the first prosthetic component.

The guide device comprises a central body able to be positioned against at least the upper lateral part of a mandibular condylar branch, and a guide wall associated at the upper part with the central body and having the profile of the osteotomy to be performed on the condyle.

According to a characteristic aspect of the present invention, the guide device has a groove which extends vertically from the guide wall toward the central body. Furthermore, the groove is open at the upper part and is through in a direction parallel to the latero-medial axis.

Some embodiments of the present invention comprise a prosthetic assembly, advantageously customized and patient-specific, provided with the prosthetic device and the corresponding guide device as above.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a perspective view of a prosthetic device for temporomandibular joint in accordance with the embodiment described here;
- fig. 2 is a perspective view of the first prosthetic component of the prosthetic device of fig. 1;
- fig. 3 is a perspective view of the second prosthetic component of the prosthetic device of fig. 1;
- fig. 4 is a lateral view of the anchoring element of fig. 2;
- fig. 5 is a perspective view showing the coupling of the first prosthetic component with the mandibular condyle;
- fig. 6 is an external lateral plan view of the first prosthetic component during use;
- fig. 7 is an internal lateral plan view of the first prosthetic component during use;
- fig. 8 is a front view of the first prosthetic component during use;
- fig. 9 is a perspective view of a guide device in accordance with the embodiment described here;
- figs. 10-11 are perspective views that show the functioning of the guide device of fig. 9.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the possible embodiments of the invention, of which one or more examples are shown in the attached drawings. Each example is supplied by way of illustration of the invention and shall not be understood as a limitation thereof. For example, one or more characteristics shown or described insomuch as they are part of one embodiment can be varied or adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

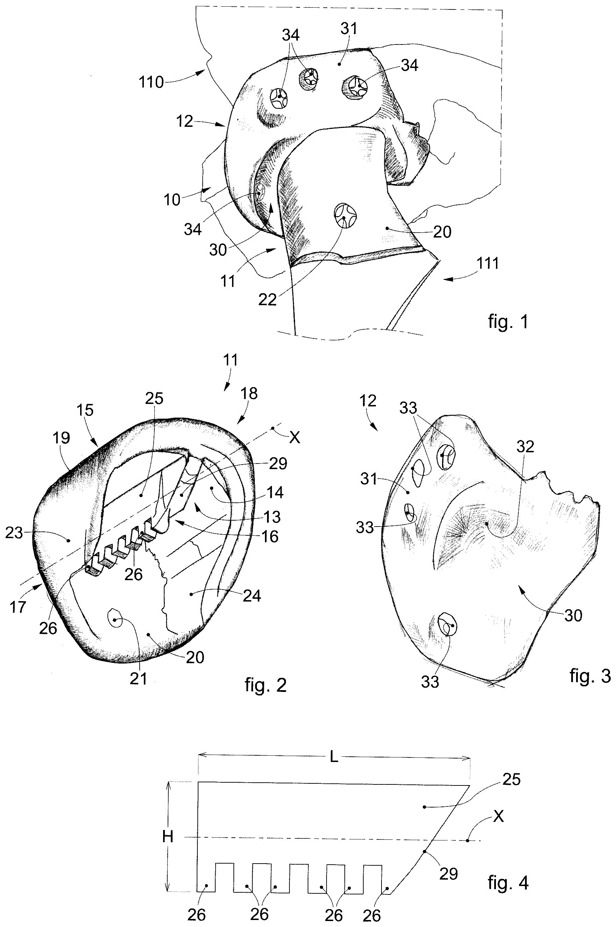

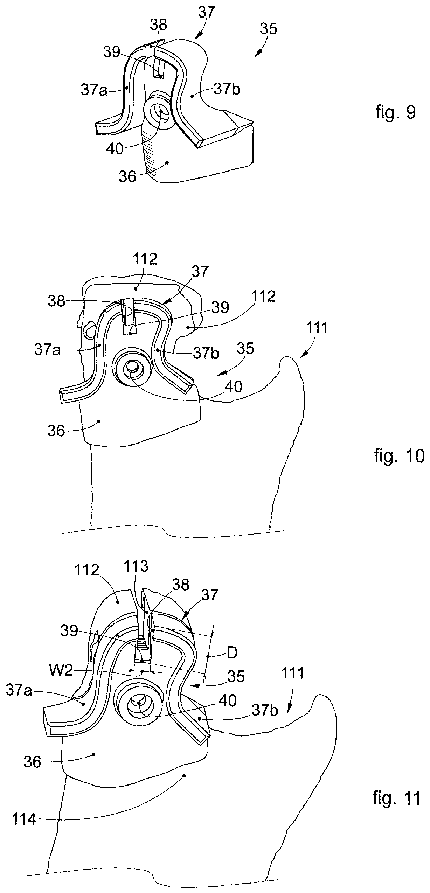

Some embodiments described here concern a prosthetic device 10 for temporomandibular joint (figs. 1-7) and a guide device 35 (figs. 9-11) required to prepare a specific anchoring seating 113 (fig. 11) for the prosthetic device 10, as will be described in more detail below. Other embodiments also include a prosthetic assembly, advantageously customized/individualized and patient-specific, which comprises the prosthetic device 10 and the guide device 35.

Here and hereafter, the terms proximal, distal, anterior, posterior, medial, lateral are defined by their standard use for a person of skill in the art to indicate a particular aspect or orientation of a bone, an anatomical part, a prosthetic device and its components, or other elements according to the relative disposition of the natural anatomy of the human and/or animal body, or directional terms of reference with respect thereto.

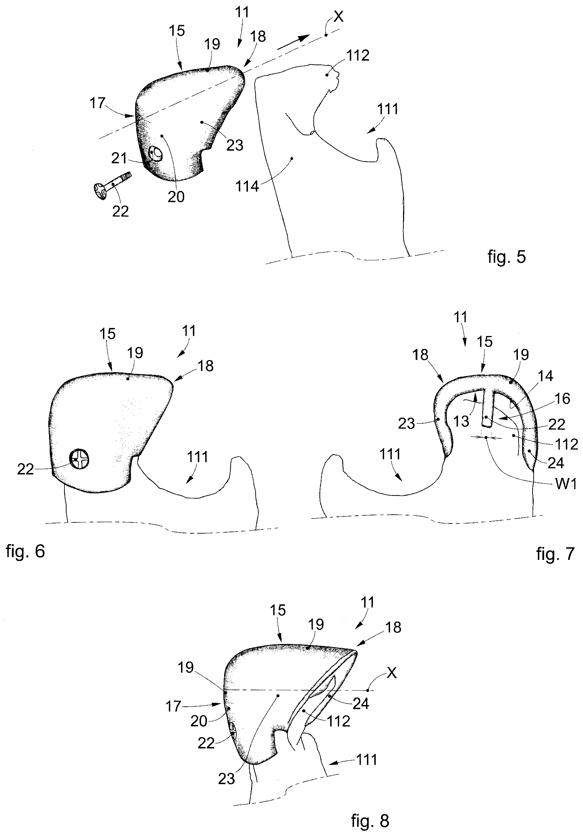

With particular reference to fig. 1, the temporomandibular joint articulates the mandibular bone 111 with the temporal bone 110, in particular it connects a mandibular condyle (fig. 5), hereafter condyle, 112 with a respective glenoid fossa of the temporal bone 110, hereafter fossa.

The prosthetic device 10 therefore comprises a first prosthetic component 11, able to be associated with a condyle 112 of a patient, and a coordinated second prosthetic component 12, able to be associated with a respective fossa of the patient. In particular, the condyle 112 is suitably shaped to house the first prosthetic component 11, as will be described in more detail below.

The first prosthetic component 11 comprises a concave portion 13, defining a coupling seating 14 having a shape mating with the shape of the previously shaped condyle 112.

The first component 11 comprises a convex portion 15, opposite the concave portion 13 and configured to couple with the second prosthetic component 12 to define the temporomandibular joint.

The first prosthetic component 11 is provided with at least one anchoring element 16 projecting from, and attached at least to, the concave portion 13. The anchoring element 16 has an overall size such as to be substantially contained inside the concave portion 13.

The anchoring element 16 has a main extension along a latero-medial axis X. In particular, the term latero-medial here and hereafter is intended to indicate a direction that goes from a lateral zone toward a zone located in the proximity of the median plane of the patient's body.

The anchoring element 16 is configured to be inserted in a mating anchoring seating 113 (fig. 11) made on the condyle 112 of the patient which is shaped following the profile of the guide device 35 which will be described below.

The coupling seating 14 is at least partly open along the latero-medial axis X so as to allow, in one possible application, the coupling of the first prosthetic component 11 with the condyle 112 in a direction parallel to the latero-medial axis, which runs from the outside to the inside (fig. 2, fig. 5 and fig. 7-8). In the embodiment described here, the coupling seating 14 has a shape substantially mating with the articulating upper portion of the condyle 112.

The particular geometry of the prosthetic device 10 and in particular of the first prosthetic component 11 provided with the anchoring element 16 with latero-medial insertion allows to limit the removal of bone material from the patient to a minimum, and at the same time allows to greatly simplify the surgical practice which becomes less invasive thanks to the need to only create one access route, with a pre-aural incision.

According to some embodiments described here, with particular reference to figs. 2, 5 and 8, the first prosthetic component 11 also comprises an external lateral portion 17 configured to couple laterally with the condyle 112. The external lateral portion 17 has an extension such as to allow a lateral abutment during the positioning of the first prosthetic component 11 along the latero-medial axis X, and to allow a stabilization thereof.

The first prosthetic component 11 can be configured as a condyle prosthesis configured to at least partly cover the condyle 112 of the patient. This first prosthetic component 11 is conceived as a covering prosthesis with the purpose of preserving the original bone portion of the condyle 112 as much as possible. Favorably, this condylar prosthesis can be used in the case of non-extensive pathologies, which in fact are those with the greatest incidence.

In particular, the first prosthetic component 11 is configured to cover the lateral surface of the condyle 112, the upper surface - the one that normally articulates with the fossa - and possibly also the posterior and anterior surfaces. Obviously, given the mode of insertion of the first prosthetic component 11, that is, in a direction parallel to the latero-medial axis X, it is not provided to cover the medial portion of the condyle 112 (figs. 7-8).

With particular reference to fig. 2, the first prosthetic component 11 comprises a shell 18 having an upper wall 19 and an external lateral wall 20 disposed inclined with respect to the upper wall 19. The inclination of the external lateral wall 20 is essentially given by the anatomy of the mandibular branch 114 of the patient. In the example embodiment described here, this inclination is about 90°, or slightly smaller.

The upper wall 19 has a shape such as to define, at the bottom, the concave portion 13 and, at the upper part, the convex portion 15 (fig. 2 and fig. 7).

The upper wall 19 is configured to at least partly cover the condyle leaving an internal medial part thereof substantially free. For this purpose, the shell 18 is open, as well as at the bottom, also along the latero-medial axis X in a zone opposite the external lateral wall 20 and terminal of the upper wall 19 (figs. 7-8).

The external lateral wall 20 has a main development along the mandibular branch 114 and is substantially parallel or subparallel to the median plane of the body.

Optionally, the external lateral wall 20 can be provided with at least one through hole 21 for the insertion of an attachment element, for example a screw 22 (see for example fig. 2 and figs 5-6). The screw 22 allows to obtain a primary stabilization of the first prosthetic component 11 at least during the period of osseointegration thereof with the mandibular condyle of the patient. The size and length of the screw 22 vary according to the conformation and sizes of the condyle.

In the embodiment described here, with particular reference to fig. 2 and fig. 7, the shell 18 also comprises a pair of opposite walls, respectively an anterior wall 23 and a posterior wall 24, configured to at least partly wrap the condyle anteriorly and posteriorly in order to guarantee greater stability. The anterior 23 and posterior wall 24 are connected at the upper part to the upper wall 19, respectively on one side and the other of the latter, and laterally to the external lateral wall 20.

Although the upper wall 19, the external lateral wall 20 and the anterior 23 and posterior walls 24 have been described as distinct and separate elements, it goes without saying that they can be made in a single body so that one can be an extension of the other, according to the geometry and the reciprocal disposition just described.

According to some embodiments described here, the anchoring element 16 is configured as a lamella 25 attached at the upper part to the upper wall 19, on the side of the concave portion 13, and laterally to the external lateral wall 20. However, it is not excluded that the lamella 25 can be attached only to the upper part 19. The lamella 25 can be conformed as a wall, a septum, a suitably shaped ridge projecting from the upper wall 19.

In particular, the lamella 25 projects in a direction substantially orthogonal to the upper wall 19 (fig. 2 and fig. 7). The lamella 25 divides the coupling seating 14 substantially into two parts, each of which is able to rest on a respective portion of condyle 112 located on the sides of the anchoring seating 113 (fig. 11).

The lamella 25 has a longitudinal extension, or length, L (fig. 4) in the direction of the latero-medial axis X substantially equal to the extension of the upper wall 19 in the same direction (fig. 2).

However, it is not excluded that the lamella 25 may also have a shorter length L. Evidently, the greater the length L, the greater the useful surface in contact with the bone of the condyle for a better integration and stabilization.

The lamella 25 has a height H (fig. 4) proportional to the depth of the anchoring seating 113 (fig. 11) provided on the condyle 112. For example, the height H can have a minimum value of 4 mm, or be larger.

The lamella 25 has a thickness W1 (fig. 7) much smaller than the length L. For example, the thickness W1 can vary between about 1mm and about 4mm. Favorably, the thickness W1 is comprised between about 2mm and about 3mm.

Advantageously, the lamella 25 is configured to promote the primary stabilization of the prosthesis, the neoformation of cancellous bone and the osseointegration with the cortical bone of the condyle 112.

In particular, the lamella 25 can have a substantially rectangular shape and be provided with macro grooves and micro grooves to promote the neoformation of cancellous bone and the osseointegration with the cortical bone of the condyle 112.

In one embodiment, fig. 2 and 4, the lamella 25 is provided with a plurality of teeth 26 suitably inter-spaced to define the macro grooves as above, in such a way as to promote, during use, bone growth inside them in order to guarantee a greater stability of the first prosthetic component 11. For example, the lamella 25 and the teeth 26 define a comb-like conformation.

The lamella 25 can also be provided with a chamfer 29 able to facilitate the latero-medial insertion of the first prosthetic component 11 in the anchoring seating 113 on the condyle 112. In this case, fig. 4, the shape of the lamella 25 can be a rectangle trapezoid in which the inclined side that defines the chamfer 29 can also have more than one inclination or be defined by a curve.

According to some embodiments, the first prosthetic component 11 is made with a biocompatible material, for example titanium or an alloy thereof, or other possible known or unknown biocompatible materials. Advantageously, the articulating surface of this first prosthetic component 11 can be treated so that it is as smooth as possible, for example mirror-like, in order to reduce frictions during joint movement to a minimum.

In particular, the lamella 25 can be made of titanium, and its surface can be favorably treated to increase the contact surface with the bone and promote osseointegration with the latter. For example, the surface of the lamella 25 can be treated with a sandblasting process with hydroxyapatite and acid passivation (RBM). In possible embodiments, the lamella 25 can, alternatively and for the purposes of osseointegration, have an at least partly porous or lattice-shaped structure, which for example reproduces the trabecular structure of the bone, possibly associated with a portion of compact material.

The best osseointegration is also promoted by the piezo surgical preparation of the surgical site, which provides a preservation of the bone tissues and much less damage than a traditional cutter for the preparation of an osteotomy site.

In some embodiments, the first prosthetic component 11 can be made with a Selective Laser Melting (SLM) process, or with a Direct Metal Laser Sintering (DMSL) process, or again by means of Electron Beam Melting (EBM) technique, or in general by means of a suitable "additive manufacturing" or 3D printing technique, based on the specific anatomical needs of the patient. Such techniques, for example, are advantageous in the event the first prosthetic component 11, and in particular the lamella 25, at least partly have an at least partly porous or lattice-shaped structure, for example trabecular, possibly associated with a portion of compact material.

According to the embodiment shown in fig. 1 and fig. 3, the second prosthetic component 12 can be configured as a fossa prosthesis configured to cover the glenoid fossa of the patient.

The second prosthetic component 12 comprises an articulating portion 30 able to cooperate, during use, with the convex portion 15 of the first prosthetic component 11, and a zygomatic portion 31 which, during use, is able to cover the zygomatic arch of the patient.

The articulating portion 30 has a convex articulating surface 32 having a shape mating with the convex portion 15.

The upper part of the articulating portion 30, the one which, during use, is positioned in contact with the glenoid fossa of the patient, has a shape that traces the pre-existing, or surgically shaped, bone surface of the fossa.

The second prosthetic component 12 can be stabilized to the temporal bone by means of screws 34. For this purpose, both the articulating portion, in an outermost part thereof, and also the zygomatic portion 31 can provide through holes 33 for the screws 34. In the example described here, the zygomatic portion 31 is stabilized with three screws 34.

According to some embodiments, the second prosthetic component 12 is made with a biocompatible material, for example ultra-high molecular weight polyethylene (UHMWPE), or other possible known and unknown biocompatible materials.

In accordance with the embodiment described in figs. 9-11, the guide device, in this specific case a surgical template, 35 comprises a central body 36 able to wrap at least the upper lateral part of the condylar branch, and a guide wall 37 attached at the upper part to the central body 36 and having the profile of the osteotomy to be performed on the condyle 112 of the patient. In particular, the central body 36 can be suitable to also partly wrap the posterior and anterior surfaces of the condyle 112 for a better positioning.

The surgical template 35 has a central vertical groove 38 open at the upper part which divides the guide wall 37 into a first guide branch 37a and a second guide branch 37b.

In the example described here, the guide wall 37 has a substantially curved profile, however, it is not excluded that the guide wall 37 may have a squared profile, or other profile suitable to make the least invasive osteotomy possible.

The vertical groove 38 is through in a direction parallel to the latero-medial axis X in order to allow the surgeon to perform a vertical osteotomy in order to prepare the anchoring seating 113 in the condyle 112 of the patient.

The guide groove 38 can also extend, in part, in the central body 36.

The groove 38 is open at the upper part and has a terminal part, or bottom, 39 able to act as an abutment for the surgical blade during the preparation of the anchoring seating 113 (fig. 11).

The groove 38 has a depth D substantially equal to the height H of the lamella 25 of the first prosthetic component 11, and a width W2 smaller than or equal to the thickness W1 of the lamella 25 (fig. 11). Furthermore, it is possible to provide a slight tapering that goes from the upper aperture of the groove 38 to the terminal part 39 thereof.

The surgical template 35 is provided with at least one calibrated hole 40 prepared through the central body 36. The calibrated hole 40 allows to prepare a corresponding hole in the condyle 112 of the patient to position a screw which serves to stabilize the surgical template 35 and subsequently to attach the first prosthetic component 11. In possible implementations, the condylar prosthetic component, that is, the condyle 112, can be modeled and shaped in its external lateral part in order to house more attachment screws and, for this purpose, this can also provide the use of a dedicated template.

Operatively, after the identification of the part of the condyle 112 to be removed, the surgical template 35, having profiles suitably shaped according to the anatomical needs of the patient, is positioned on the condylar branch 114 and attached to it with a screw (not shown) (fig. 10). Subsequently, a first osteotomy is performed following the profile of the guide wall 37 and then a second vertical osteotomy is performed along the groove 38 to create the anchoring seating 113 (fig. 11) to house the lamella 25. Favorably, the osteotomies are performed using a blade with piezoelectric handle, guaranteeing maximum cutting precision.

It is clear that modifications and/or additions of parts may be made to the prosthetic device for temporomandibular joint and corresponding prosthetic assembly as described heretofore, without departing from the field and scope of the present invention as defined by the claims.

It is also clear that, although the present invention has been described with reference to some specific examples, a person of skill in the art shall certainly be able to achieve many other equivalent forms of prosthetic device for temporomandibular joint and corresponding prosthetic assembly, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Prosthetic device (10) for temporomandibular joint comprising a first prosthetic component (11), able to be associated with a mandibular condyle (112) of a patient, and a coordinated second prosthetic component (12), able to be associated with a respective glenoid fossa of said patient, wherein said first prosthetic component (11) is configured to cooperate with said second prosthetic component (12) to define said temporomandibular joint and comprises a concave portion (13), defining a coupling seating (14) having a shape mating with the shape of said mandibular condyle, **characterized in that** said first prosthetic component (11) comprises at least one anchoring element (16) projecting from, and attached at least to, said concave portion (13), being contained in said concave portion (13), said anchoring element (16) having a main extension along a latero-medial axis (X) and being configured to be inserted into a mating anchoring seating (113) present on said mandibular condyle (112), **and in that** said coupling seating (14) is at least partly open along said latero-medial axis (X).

2. Prosthetic device as in claim 1, **characterized in that** said first prosthetic component (11) further comprises an external lateral portion (17) configured to couple laterally with said condyle (112).

3. Prosthetic device as in claim 1 or 2, **characterized in that** said first prosthetic component (11) comprises a shell (18) having an upper wall (19) and an external lateral wall (20), disposed inclined with respect to said upper wall (19), wherein said shell (18) is open, as well as at the bottom, also along said latero-medial axis (X) in a zone opposite said external lateral wall (20) and terminal of said upper wall (19).

4. Prosthetic device as in claim 3, **characterized in that** said anchoring element (16) is configured as a lamella (25) fixed at the upper part to said upper wall (19), on the side of said concave portion (13), and laterally to said external lateral wall (20), said lamella (25) projecting in a direction substantially orthogonal to said upper wall (19).

5. Prosthetic device as in claim 4, **characterized in that** said lamella (25) has a longitudinal extension, or length, (L) in the direction of said latero-medial axis (X) substantially equal to the extension of said upper wall (19) in the same direction.

6. Prosthetic device as in claim 5, **characterized in that** said lamella (25) is configured to promote the primary stabilization of the prosthesis, the neoformation of cancellous bone and the osseointegration with the cortical bone of said condyle (112).

7. Prosthetic device as in claim 6, **characterized in that** said lamella (25) is provided with macro grooves and micro grooves to promote the neoformation of cancellous bone and the osseointegration with the cortical bone of said condyle (112).

8. Prosthetic device as in claim 7, **characterized in that** said lamella (25) is provided with a plurality of teeth (26) suitably inter-spaced to define said macro grooves.

9. Guide device for preparing an anchoring seating (113) for a prosthetic device as in any claim from 4 to 8, comprising a central body (36) able to be positioned against at least the upper lateral part of a mandibular condylar branch, and a guide wall (37) associated at the upper part with said central body (36) and having the profile of the osteotomy to be performed on said condyle (112), **characterized in that** it has a groove (38) which extends vertically from said guide wall (37) toward said central body (36), **and in that** said groove (38) is open at the upper part and is through in a direction parallel to a latero-medial axis (X), **and in that** said groove (38) has a depth (D) substantially the same as a height (H) of a lamella (25) which acts as an anchoring element (16) of said prosthetic device and a width (W2) being smaller than or equal to the thickness (W1) of the lamella (25).

10. Guide device as in claim 9, **characterized in that** said groove (38) divides said guide wall (37) into a first guide branch (37a) and a second guide branch (37b).

11. Guide device as in claim 9 or 10, **characterized in that** said groove (38) has a terminal part, or bottom (39), able to act as an abutment for a surgical blade.

12. Prosthetic assembly for temporomandibular joint comprising a prosthetic device (10) as in any claim from 1 to 8 and a guide device (35) as in any claim from 9 to 11.

## Patentansprüche

1. Prothesenvorrichtung (10) für das Kiefergelenk, aufweisend eine erste Prothesenkomponente (11), die mit einem Unterkiefer-Kondylus (112) eines Patienten verbunden werden kann, und eine abgestimmte zweite Prothesenkomponente (12), die mit einer betreffenden Gelenkpfanne des Patienten verbunden werden kann, wobei die erste Prothesenkomponente (11) so konfiguriert ist, dass sie mit der zweiten Prothesenkomponente (12) zusammenwirken kann, um das Kiefergelenk zu definieren, und einen konkaven Abschnitt (13) aufweist, der einen Kupplungssitz (14) definiert, dessen Form mit der Form des Unterkiefer-Kondylus zusammenpasst, **dadurch gekennzeichnet, dass** die erste Prothesenkomponente (11) mindestens ein Verankerungselement (16) aufweist, das aus dem konkaven Abschnitt (13) hervorsteht und zumindest an diesem befestigt ist und in dem konkaven Abschnitt (13) aufgenommen ist, wobei das Verankerungselement (16) eine Haupterstreckung entlang einer latero-medialen Achse (X) hat und konfiguriert ist, um in einen passenden Verankerungssitz (113) eingesetzt zu sein, der an dem Unterkiefer-Kondylus (112) vorhanden ist, **und dass** der Kupplungssitz (14) entlang der latero-medialen Achse (X) zumindest teilweise offen ist.

2. Prothesenvorrichtung nach Anspruch 1, **dadurch gekennzeichnet, dass** die erste Prothesenkomponente (11) ferner einen äußeren lateralen Abschnitt (17) aufweist, der konfiguriert ist, um lateral mit dem Kondylus (112) gekoppelt zu sein.

3. Prothesenvorrichtung nach Anspruch 1 oder 2, **dadurch gekennzeichnet, dass** die erste Prothesenkomponente (11) eine Schale (18) aufweist, die eine obere Wand (19) und eine äußere laterale Wand (20) hat, die in Bezug auf die obere Wand (19) geneigt angeordnet ist, wobei die Schale (18) offen ist sowohl am Boden als auch entlang der latero-medialen Achse (X) in einem Bereich entgegengesetzt der äußeren lateralen Wand (20) und am Ende der oberen Wand (19) .

4. Prothesenvorrichtung nach Anspruch 3, **dadurch gekennzeichnet, dass** das Verankerungselement (16) als eine Lamelle (25) ausgebildet ist, die am oberen Teil an der oberen Wand (19) auf der Seite des konkaven Abschnitts (13) und lateral an der äußeren lateralen Wand (20) befestigt ist, wobei die Lamelle (25) in einer Richtung im Wesentlichen orthogonal zur oberen Wand (19) vorsteht.

5. Prothesenvorrichtung gemäß Anspruch 4, **dadurch gekennzeichnet, dass** die Lamelle (25) eine Längserstreckung oder Länge (L) in Richtung der latero-medialen Achse (X) hat, die im Wesentlichen gleich der Erstreckung der oberen Wand (19) in gleichen Richtung ist.

6. Prothesenvorrichtung nach Anspruch 5, **dadurch gekennzeichnet, dass** die Lamelle (25) so konfiguriert ist, dass sie die primäre Stabilisierung der Prothese, die Neubildung von spongiösem Knochen und die Osseointegration mit dem kortikalen Knochen des Kondylus (112) fördert.

7. Prothesenvorrichtung nach Anspruch 6, **dadurch gekennzeichnet, dass** die Lamelle (25) mit Makrorillen und Mikrorillen versehen ist, um die Neubildung von spongiösem Knochen und die Osseointegration mit dem Kortikalknochen des Kondylus (112) zu fördern.

8. Prothesenvorrichtung nach Anspruch 7, **dadurch gekennzeichnet, dass** die Lamelle (25) mit einer Vielzahl von Zähnen (26) versehen ist, die in geeigneten Abständen voneinander angeordnet sind, um die Makrorillen zu definieren.

9. Führungsvorrichtung zum Vorbereiten eines Verankerungssitzes (113) für eine Prothesenvorrichtung nach irgendeinem Anspruch von 4 bis 8, aufweisend einen zentralen Körper (36), der gegen mindestens den oberen lateralen Teil eines Unterkiefer-Kondylus-Zweigs positioniert werden kann, und eine Führungswand (37), die im oberen Teil mit dem zentralen Körper (36) verbunden ist und das Profil der an dem Kondylus (112) durchzuführenden Osteotomie hat, **dadurch gekennzeichnet, dass** sie eine Nut (38) hat, die sich vertikal von der Führungswand (37) aus zum zentralen Körper (36) hin erstreckt, **und dass** die Nut (38) im oberen Teil offen ist und in einer Richtung parallel zu einer latero-medialen Achse (X) durchgehend ist, **und dass** die Nut (38) eine Tiefe (D), die im Wesentlichen gleich einer Höhe (H) einer Lamelle (25) ist, die als ein Verankerungselement (16) der Prothesenvorrichtung dient, und eine Breite (W2) hat, die kleiner oder gleich der Dicke (W1) der Lamelle (25) ist.

10. Führungsvorrichtung nach Anspruch 9, **dadurch gekennzeichnet, dass** die Nut (38) die Führungswand (37) in einen ersten Führungszweig (37a) und einen zweiten Führungszweig (37b) unterteilt.

11. Führungsvorrichtung nach Anspruch 9 oder 10, **dadurch gekennzeichnet, dass** die Nut (38) einen Endteil oder Boden (39) hat, der imstande ist, als ein Anschlag für eine chirurgische Klinge zu dienen.

12. Prothesenbaugruppe für das Kiefergelenk, aufweisend eine Prothesenvorrichtung (10) nach irgendeinem Anspruch von 1 bis 8 und eine Führungsvorrichtung (35) nach irgendeinem Anspruch von 9 bis 11.

## Revendications

1. Dispositif prothétique (10) pour articulation temporo-mandibulaire comprenant un premier composant prothétique (11), pouvant être associé à un condyle mandibulaire (112) d'un patient, et un second composant prothétique coordonné (12), pouvant être associé à une fosse glénoïdienne respective dudit patient, dans lequel ledit premier composant prothétique (11) est configuré pour coopérer avec ledit second composant prothétique (12) pour définir ladite articulation temporo-mandibulaire et comprend une partie concave (13), définissant un siège de couplage (14) ayant une forme correspondant à la forme dudit condyle mandibulaire, **caractérisé en ce que** ledit premier composant prothétique (11) comprend au moins un élément d'ancrage (16) faisant saillie à partir de, et fixé au moins à, ladite partie concave (13), étant contenu dans ladite partie concave (13), ledit élément d'ancrage (16) ayant une extension principale le long d'un axe latéro-médial (X) et étant configuré pour être inséré dans un siège d'ancrage correspondant (113) présent sur ledit condyle mandibulaire (112), **et en ce que** ledit siège de couplage (14) est au moins partiellement ouvert le long dudit axe latéro-médial (X).

2. Dispositif prothétique selon la revendication 1, **caractérisé en ce que** ledit premier composant prothétique (11) comprend en outre une partie latérale externe (17) configurée pour se coupler latéralement avec ledit condyle (112).

3. Dispositif prothétique selon la revendication 1 ou 2, **caractérisé en ce que** ledit premier composant prothétique (11) comprend une coque (18) ayant une paroi supérieure (19) et une paroi latérale externe (20), disposée inclinée par rapport à ladite paroi supérieure (19), dans lequel ladite coque (18) est ouverte, ainsi qu'au fond, également le long dudit axe latéro-médial (X) dans une zone opposée à ladite paroi latérale externe (20) et au terminal de ladite paroi supérieure (19).

4. Dispositif prothétique selon la revendication 3, **caractérisé en ce que** ledit élément d'ancrage (16) est configuré comme une lamelle (25) fixée au niveau de la partie supérieure à ladite paroi supérieure (19), du côté de ladite partie concave (13), et latéralement à ladite paroi latérale externe (20), ladite lamelle (25) faisant saillie dans une direction sensiblement orthogonale à ladite paroi supérieure (19).

5. Dispositif prothétique selon la revendication 4, **caractérisé en ce que** ladite lamelle (25) a une extension longitudinale, ou longueur, (L) dans la direction dudit axe latéro-médial (X) sensiblement égale à l'extension de ladite paroi supérieure (19) dans la même direction.

6. Dispositif prothétique selon la revendication 5, **caractérisé en ce que** ladite lamelle (25) est configurée pour favoriser la stabilisation primaire de la prothèse, la néoformation de l'os spongieux et l'ostéointégration avec l'os cortical dudit condyle (112).

7. Dispositif prothétique selon la revendication 6, **caractérisé en ce que** ladite lamelle (25) est pourvue de macro-rainures et de micro-rainures pour favoriser la néoformation de l'os spongieux et l'ostéointégration avec l'os cortical dudit condyle (112).

8. Dispositif prothétique selon la revendication 7, **caractérisé en ce que** ladite lamelle (25) est pourvue d'une pluralité de dents (26) espacées de manière appropriée pour définir lesdites macro-rainures.

9. Dispositif de guidage pour préparer un siège d'ancrage (113) pour un dispositif prothétique selon l'une quelconque des revendications 4 à 8, comprenant un corps central (36) pouvant être positionné contre au moins la partie latérale supérieure d'une branche condylienne mandibulaire, et une paroi de guidage (37) associée au niveau de la partie supérieure audit corps central (36) et ayant le profil de l'ostéotomie à effectuer sur ledit condyle (112), **caractérisé en ce qu'**il a une rainure (38) qui s'étend verticalement depuis ladite paroi de guidage (37) vers ledit corps central (36), **et en ce que** ladite rainure (38) est ouverte au niveau de la partie supérieure et est traversante dans une direction parallèle à un axe latéro-médial (X), **et en ce que** ladite rainure (38) a une profondeur (D) sensiblement la même qu'une hauteur (H) d'une lamelle (25) qui agit comme un élément d'ancrage (16) dudit dispositif prothétique et une largeur (W2) étant inférieure ou égale à l'épaisseur (W1) de la lamelle (25).

10. Dispositif de guidage selon la revendication 9, **caractérisé en ce que** ladite rainure (38) divise ladite paroi de guidage (37) en une première branche de guidage (37a) et une seconde branche de guidage (37b).

11. Dispositif de guidage selon la revendication 9 ou 10, **caractérisé en ce que** ladite rainure (38) a une partie terminale, ou fond (39), pouvant servir de butée pour une lame chirurgicale.

12. Ensemble prothétique pour articulation temporo-mandibulaire comprenant un dispositif prothétique (10) selon l'une quelconque des revendications 1 à 8 et un dispositif de guidage (35) selon l'une quelconque des revendications 9 à 11.
